# EUROPÄISCHE PATENTSCHRIFT

(11) **EP 0 787 175 B1**
(45) Veröffentlichungstag und Bekanntmachung des Hinweises auf die Patenterteilung: **16.09.1998**
(21) Anmeldenummer: 95935918.3
(22) Anmeldetag: 09.10.1995
(51) Int. Cl.: C11D 1/835

(54) **TEXTILE WEICHMACHER-KONZENTRATE**
CONCENTRATED TEXTILE SOFTENERS
ASSOUPLISSANTS TEXTILES CONCENTRES

(30) Priorität: 17.10.1994 DE 4437032
(43) Veröffentlichungstag der Anmeldung: 06.08.1997
(73) Patentinhaber: Henkel Kommanditgesellschaft auf Aktien, 40191 Düsseldorf (DE)
(72) Erfinder: BEHLER, Ansgar, D-46240 Bottrop (DE); UPHUES, Günther, D-40789 Monheim (DE); WAHLE, Bernd, D-41564 Kaarst (DE); WALTENBERGER, Peter, D-53547 Breitscheid (DE); FOLGE, Almud, D-40723 Hilden (DE)
(86) Internationale Anmeldenummer: EP9503969
(87) Internationale Veröffentlichungsnummer: WO9612002

(56) Entgegenhaltungen:
- EP-A- 0 243 735
- EP-A- 0 494 769
- EP-A- 0 530 959
- WO-A-92/18593
- WO-A-93/23510
- WO-A-94/06900
- WO-A-95/00614
- DATABASE WPI Section Ch, Week 9013 Derwent Publications Ltd., London, GB; Class E19, AN 90-095550 & JP,A,02 047 362 ( KAO CORP) , 16.Februar 1990

## Beschreibung

Die Erfindung liegt auf dem allgemeinen Gebiet der Textilbehandlungsmittel und betrifft textile Weichmacher-Konzentrate mit verbesserter Kaltwasserdispergierbarkeit auf Basis von Penta- und/oder Dipentaerythritfettsäurepartialestern und einer speziellen Emulgatorkombination aus bestimmten quaternären Stickstoffverbindungen und bestimmten nicht-ionogenen Verbindungen, ein Verfahren zur ihrer Herstellung und die Verwendung des Emulgatorsystems.

Unter textilen Weichmacher-Konzentraten werden im Rahmen dieser Erfindung Mittel verstanden, die sowohl in der Textiltechnik zur Veredlung von Garnen, Fasern, Gewirken, Geweben oder Nonwovens als auch in Nachbehandlungsmitteln von gewaschenen Textilien im Haushalt eingesetzt werden können.

Weichmacher werden in der Textiltechnik und in Nachbehandlungsmitteln von gewaschenen Textilien zur Veredlung eingesetzt, um beispielsweise einen angenehmen flauschigen Griff zu erzeugen, den Tragekomfort zu erhöhen, die Verarbeitungseigenschaften oder auch die Pflege der Textilien zu verbessern. Üblicherweise werden als Weichmacher quaternäre Ammoniumverbindungen eingesetzt, da sie besonders leicht auf Textilien aufziehen. Ein Beispiel für derartige quaternäre Ammoniumverbindungen sind quaternierte Difettsäurealkanolaminester, sogenannte Esterquats, die jedoch in höheren Konzentrationen in kaltem Wasser ein unzureichendes Auflösungs- bzw. Dispergiervermögen zeigen, wodurch beispielsweise schon die Herstellung von wäßrigen Konzentraten mit einem Aktivsubstanzgehalt an Esterquats unter 30 Gew.-% erschwert wird. Eine Verbesserung der Kaltwasserdispergierbarkeit kann gemäß DE-A-36 12 479 durch Zusatz geringer Mengen an Carbonsäureester oder gemäß DE-A-42 32 448 durch Zusatz von Fettalkoholen, Fettalkoholpolyglykolether oder Polyolfettsäurepartialester erfolgen.

In jüngster Zeit sind anstelle von Esterquats oder anderen quaternären Ammoniumverbindungen Ester des Penta- und/oder Dipentaerythrits als Weichmacher vorgeschlagen worden. So beschreibt beispielsweise die EP-A-494 769 wässrige textile Weichmacher, die als Weichmacherhauptkomponente insbesondere Pentaerythritdistearat enthalten. Geringe Mengen an quaternären Ammoniumverbindungen als Weichmacher wie Esterquats können jedoch zugelassen sein. Um wäßrige Emulsionen mit einem Aktivgehalt von Pentaerythritestern von 1-25 % zu erreichen, empfiehlt die EP-A-494 769 den Zusatz von Emulgatoren wie höhere Alkyldialkanolamine und/oder Alkylpolyethylenglykolether. Diese Emulgatoren sind zwar geeignet, um niedere Konzentrate mit einem Aktivgehalt von Pentaerythritestern von 1-25 % zu stabilisieren, aber bei höher gehaltigen Konzentraten verlieren sie an Effizienz. Unter Zuhilfenahme dieser Emulgatoren verfügen die wasserfreien Pentaerythrit/ Emulgator-Mischungen über eine schlechte Kaltwasserdispergierbarkeit, so daß eine Dispergierung in kaltem Wasser nur unter großem Aufwand möglich ist.

Wegen dieses Aufwandes würde der Anwender vom Hersteller die Dispergierung des Konzentrats in Wasser verlangen, was mit der Lagerung und dem Transport erheblicher Mengen Wasser verbunden und somit aus ökonomischer Sicht wenig sinnvoll ist.

Aufgabe der vorliegenden Erfindung war es daher, Emulgatoren für Weichmacherkonzentrate zur Verfügung zu stellen, die zum einen die Kaltwasserdispergierbarkeit von textilen Weichmacher-Konzentraten mit einem hohen Gehalt an Pentaerythritestern ermöglichen. Zum anderen sollten die Emulgatorzusätze nicht die Weichheitsleistung der Pentaerythritester verschlechtern und schließlich sollten die Weichmacherkonzentrate insgesamt eine gleiche Wirkung in der Weichheit erbringen können wie die aus dem Stand der Technik als sehr gut bekannten quaternären Ammoniumverbindungen.

Überraschenderweise konnte die Aufgabe gelöst werden durch eine spezielle Emulgatorkombination aus einer quaternären Ammoniumverbindung ausgewählt aus der Gruppe der quaternierten alkoxylierten Alkylamine und Esterquats und einer nicht-ionischen Verbindung ausgewählt aus der Gruppe der ethoxylierten und/oder propoxylierten Fettsäuren oder Fettsäureester und endgruppenverschlossenen Fettalkoholpolyglykolether.

Ein Gegenstand der vorliegenden Erfindung sind somit textile Weichmacher-Konzentrate mit verbesserter Kaltwasserdispergierbarkeit auf Basis von Penta- und/oder Dipentaerythritfettsäurepartialester als Weichmacher, dadurch gekennzeichnet, daß sie zusätzlich eine Emulgatorkombination enthalten aus
a) einer quaternären Stickstoffverbindung ausgewählt aus der Gruppe der
   a1) quaternierten alkoxylierten Alkylamine der Formel (I) 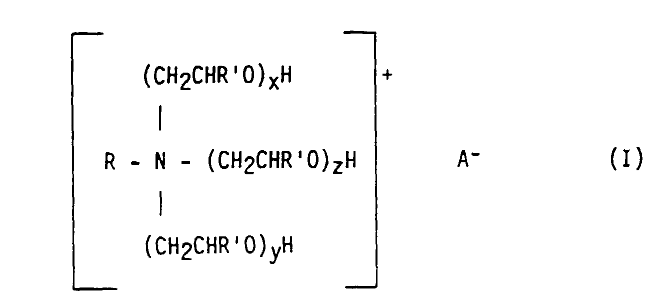 worin
      - R =: einen aliphatischen Kohlenwasserstoffrest mit 6-22 C-Atomen
      - R' =: H oder CH₃
      - x, y, z =: unabhängig voneinander eine Zahl von 1 bis 20 bedeuten, wobei die Summe x+y+z ≥ 3 ist,
      - A⁻ =: Anion bedeuten
   a2) quaternierten Fettsäurealkanolaminester der allgemeinen Formel (II) 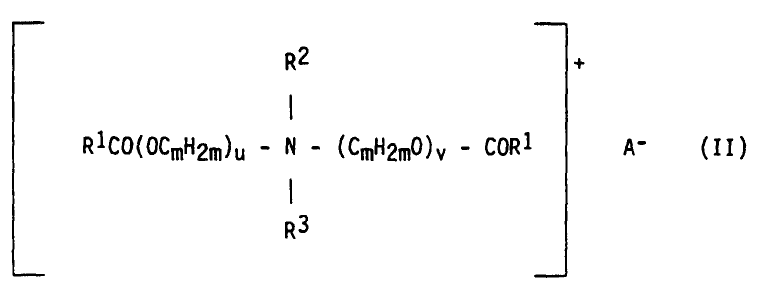 in der R¹CO - eine gesättigte oder ungesättigte Acylgruppe mit 12 bis 22 C-Atomen, R² eine Gruppe R¹CO(OCₘH₂ₘ)_{w} -, eine Alkylgruppe mit 1 bis 4 C-Atomen oder eine Hydroxyalkylgruppe mit 2 bis 4 C-Atomen und R³ eine Alkylgruppe mit 1 bis 4 C-Atomen oder eine Hydroxyalkylgruppe mit 2 bis 4 C-Atomen, m jeweils 2 oder 3, u, v und w jeweils eine Zahl von 1 bis 4 und A⁻ ein Anion ist und

   b) einem nicht-ionogenen Emulgator, der ausgewählt ist aus der Gruppe der
   b1) ethoxylierten und/oder propoxylierten Fettsäuren oder Fettsäureestern der Formel (III) 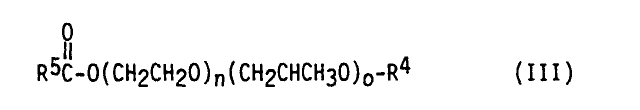 worin
      - R⁵CO =: einen aliphatischen Acylrest mit 6 bis 22 C-Atomen
      - n, o =: unabhängig voneinander eine Zahl von 0 bis 20, wobei die Summe n+o ≥ 1 ist,
      - R⁴ =: H oder einen aliphatischen Kohlenwasserstoffrest mit 1 bis 12 C-Atomen bedeuten
   b2) endgruppenverschlossenen Fettalkoholpolyglykolether der allgemeinen Formel (IV)

      R⁷-(CH₂CH₂O)ₚ(CH₂CH₃O)_{q}-R⁶ (IV),

      worin
      - R⁷ =: einen aliphatischen Kohlenwasserstoffrest mit 6 bis 22 C-Atomen,
      - p, q =: unabhängig voneinander eine Zahl von 0-20, wobei die Summe von p+q ≥ 2 ist,
      - R⁶ =: einen aliphatischen Kohlenwasserstoffrest mit 1 bis 12 C-Atomen bedeuten.

Bei den quaternierten alkoxylierten Alkylaminen der Formel (I) handelt es sich um bekannte Verbindungen, die beispielsweise gemäß DE-A-20 52 321 durch Ethoxylierung der entsprechenden Alkylamine in Gegenwart von Wasser und anschließender Neutralisation hergestellt werden können. Dabei fallen die Produkte als dünnflüssige wäßrige Lösungen an. Bevorzugt werden wäßrige Lösungen der quaternierten alkoxylierten Alkylamine der Formel (I) eingesetzt, die einen Aktivsubstanzgehalt von 40 bis 60 Gew.-% aufweisen. Die wäßrigen Lösungen werden vorzugsweise auf einen pH-Wert von 6 bis 8 eingestellt.

Sofern zur Neutralisation Phosphorsäure eingesetzt wird, steht A⁻ in Formel (I) für einen entsprechenden Äquivalentanteil des Phosphats. Aufgrund des eingestellten pH-Wertes ist anzunehmen, daß das Anion H₂PO₄-und/oder HPO₄²⁻ ist.

Innerhalb der Gruppe der quaternierten alkoxylierten Alkylamine der Formel (I) werden solche bevorzugt, in der R für einen aliphatischen Kohlenwasserstoffrest mit 12 bis 22 C-Atomen steht und x, y, z unabhängig voneinander eine Zahl von 1 bis 10 bedeuten, wobei die Summe von x+y+z im Bereich von 3 bis 10 liegt. Besonders geeignet sind die quaternierten ethoxylierten Alkylamine (R'=H).

Bei den quaternierten Fettsäurealkanolaminestern der allgemeinen Formel (II) handelt es sich ebenfalls um bekannte Verbindungen, die nach den einschlägigen Methoden der präparativen Chemie erhalten werden können. Ein Verfahren zu ihrer Herstellung beschreibt die W0 91/01295, wonach Fettsäuren mit Triethanolamin in Gegenwart von Reduktionsmitteln und unter Durchleiten von Luft umgesetzt und die erhaltenen Diester anschließend mit Alkylierungsmittel wie Dimethylsulfat, Dimethylphosphat oder Methylhalogenid quaterniert werden. Da es sich um technische Produkte handelt, sind die Esterquats der allgemeinen Formel (II) stets Mischungen von quaternierte Mono-, Di- und Triester.

Falls gewünscht können die Esterquats in Form von organischen Lösungen eingesetzt werden, beispielsweise gelöst in einem verzweigten niederen Alkohol wie Isopropanol.

Bevorzugt im Sinne der Erfindung werden quaternierte Fettsäurealkanolaminester der allgemeinen Formel (II), in der R¹CO für eine Acylgruppe steht, die sich von reinen Fettsäuren oder technischen Mischungen von Fettsäuren wie Laurin-, Myristin-, Palmitin-, Stearin-, Öl-, Elaidin-, Petroselin-, Linol-, Linolen-, Arachin-, Behen- und/oder Erucasäure ableitet. Ganz besonders bevorzugt steht in der Formel (II) R¹CO für eine gesättigte Acylgruppe mit 16 und/oder 18 C-Atomen, R³ für eine Methylgruppe, m für die Zahl 2, u, v und w für die Zahl 1 und A⁻ für ein Halogenid, Methosulfat oder Methophosphat.

Ethoxylierte und/oder propoxylierte Fettsäuren oder Fettsäureester sind ebenfalls bekannte Verbindungen, die durch Ethoxy- und/oder Propoxylierung der Fettsäuren oder Fettsäureester in Gegenwart von Katalysatoren erhalten werden. Aus der DE-A-40 10 606 ist ein Verfahren bekannt, wonach ethoxylierte und/oder propoxylierte Fettsäureester mit enger Homologenverteilung erhalten werden, sofern in Gegenwart von hydrophobierten Hydrotalciten als Katalysatoren gearbeitet wird. Es ist aber auch durchaus möglich, zunächst ethoxylierte und/oder propoxylierte Alkohole herzustellen und anschließend mit Fettsäuren zu verestern.

Je nach Anzahl der C-Atome im Alkohol- und Fettsäurerest sowie dem Alkoxylierungsgrad fallen die Produkte in flüssiger oder fester Form an.

Besonders bevorzugt aus dieser Gruppe werden die reinen Ethoxylate (o = 0) und innerhalb dieser Gruppe wiederum solche, für die in der Formel (III) R⁵CO für einen aliphatischen Acylrest mit 12 bis 22 C-Atomen und R⁴ für H oder einen Alkylrest mit 1 bis 4 C-Atomen stehen und n eine Zahl von 5 bis 15 und o die Zahl 0 bedeutet.

Beispiele für Fettsäuren, von denen sich R⁵CO ableiten kann, sind im Zusammenhang mit den Esterquats der Formel (II) beschrieben worden.

Bei den endgruppenverschlossenen Fettalkoholpolyglykolethern der allgemeinen Formel (IV) handelt es sich ebenso um eine bekannte Verbindungsklasse, die nach einschlägigen Methoden der organischen Chemie, beispielsweise durch Umsetzung von Alkylhalogeniden mit ethoxylierten und/oder propoxylierten Fettalkoholen in Anwesenheit eines Katalysators, hergestellt werden kann.

Bevorzugt im Sinne der Erfindung werden solche Verbindungen der Formel (IV), in der R⁷ für einen aliphatischen Kohlenwasserstoffrest mit 12 bis 22 C-Atomen, p für eine Zahl von 2 bis 8, q für eine Zahl von 0 bis 5 und R⁶ für einen Alkylrest mit 1 bis 4 C-Atomen steht. R⁷ kann sich von reinen Fettalkoholen oder technischen Mischungen von Fettalkoholen wie Lauryl-, Myristyl-, Palmityl-, Stearyl-, Arachidyl-, Behenyl-, Oleyl-, Elaidyl-, Linoleyl- und/oder Linolenylalkohol ableiten.

Die erfindungsgemäßen textilen Weichmacher-Konzentrate enthalten als eigentlich für die Weichheit verantwortliche Hauptkomponente Penta- und/oder Dipentaerythritfettsäurepartialester. Derartige Penta- und/oder Dipentaerythritfettsäurepartialester sind im einzelnen aus der schon zitierten EP-A-0 494 769 für diesen Anwendungszweck beschrieben. Die dort beschriebenen Penta- und Dipentaerythritfettsäurepartialester sollen im Sinn der vorliegenden Erfindung mit zum Offenbarungsgehalt der vorliegenden Anmeldung gezählt werden. Aus dieser Gruppe werden besonders Pentaerythritdifettsäureester von C₁₆₋₂₂-Fettsäuren und insbesondere reines oder technisches Pentaerythritdistearat bevorzugt.

Um die Kaltwasserdispergierbarkeit der Weichmacher-Konzentrate zu verbessern, wird den Penta- und/oder Dipentaerythritfettsäurepartialestern eine Emulgatorkombination der beschriebenen Art zugegeben.

Es hat sich als vorteilhaft erwiesen, wenn die unter b) beschriebenen nicht-ionischen Emulgatoren mindestens im gleichen Gewichtsverhältnis, vorzugsweise jedoch in höheren Mengen im Verhältnis zu den quaternären Stickstoffverbindungen zugegeben werden. Bevorzugte Gewichtsverhältnisse von nicht-ionischen Emulgatoren zu quaternären Stickstoffverbindungen betragen 1:1 bis 3:1, vorzugsweise 1,5:1 bis 2:1.

Die erfindungsgemäßen Weichmacher-Konzentrate enthalten vorzugsweise zu 70 bis 95 Gew.-% Penta- und/oder Dipentaerythritfettsäurepartialester 5 bis 30 Gew.-% beschriebene Emulgatorkombinationen und
0 bis 20 Gew.-% übliche Hilfsstoffe.

Übliche Hilfsstoffe sind beispielsweise pH-Wert Regulatoren wie organische und anorganische Säuren, Schauminhibitoren, Viskositätsregulatoren, Antioxidantien, Farb-, Duftstoffe und Soil-Release-Wirkstoffe. Zusätzliches Wasser enthalten die erfindungsgemäßen Weichmacher-Konzentrate vorteilhafterweise nicht, da hohe Gehalte an Wirkstoffen gewünscht werden. Es versteht sich jedoch, daß etwas Wasser in den Konzentraten enthalten sein kann, das beispielsweise produktionsbedingt durch die Wirk- und Hilfsstoffe eingebracht werden kann, z. B. durch Zugabe von Salzsäure (Hilfsstoff) oder bei Zusatz der wäßrigen Lösung an Verbindungen der Formel (I). Derartige Wassermengen fallen dann auch unter die 0 bis 20 Gew.-% der Hilfsstoffe.

Die erfindungsgemäßen Weichmacher-Konzentrate zeichnen sich durch eine gute Kaltwasserdispergierbarkeit aus. Zudem verschlechtert der Zusatz der angegebenen Emulgatorkombination nicht die Leistung der Penta- und/oder Dipentaerythritfettsäurepartialester und insgesamt können Weichheitsleistungen bei der Anwendung erzielt werden, die vergleichbar sind mit dem als überragend bekannten Dimethyldistearylammoniumchlorid.

Zur Anwendung können die erfindungsgemäßen Weichmacher-Konzentrate auf die gewünschte Konzentration mit Wasser verdünnt werden. Übliche Konzentrationen für Weichspüler im Haushalt liegen bei 3 bis 6 %. Man kann aber auch höhere Konzentrate mit 10 bis 30 % textilweichmachenden Wirkstoffen herstellen und entsprechend geringere Mengen dem letzten Spülbad zusetzen.

Die erfindungsgemäßen Weichmacher-Konzentrate können nach dem Verdünnen mit Wasser auf die üblichen Konzentrationen auch in der Textiltechnik verwendet werden, da sie sich leicht durch übliche Methoden wie Auszieh-, Tauchschleuder-, Foulard- oder Sprühverfahren applizieren lassen.

Ein weiterer Gegenstand der vorliegenden Erfindung betrifft ein Verfahren zur Herstellung von textilen Weichmacher-Konzentraten, dadurch gekennzeichnet, daß Penta- und/oder Dipentaerythritfettsäurepartialester als Weichmacher zunächst aufgeschmolzen und mit einer Emulgatorkombination nach Anspruch 1 versetzt werden.

Im Sinne der Erfindung ist es unerheblich, in welcher Reihenfolge die quarternären Stickstoffverbindungen und nicht-ionischen Emulgatoren zugegeben werden.

Die erhaltenen Produkte können auf übliche Weise konfektioniert werden, beispielsweise durch Sprühkristallisation oder Walzengranulierer.

Ein weiterer Gegenstand der vorliegenden Erfindung betrifft die Verwendung einer Kombination aus
a) einer quaternären Stickstoffverbindung ausgewählt aus der Gruppe der
   a1) quaternierten alkoxylierten Alkylamine der Formel (I) 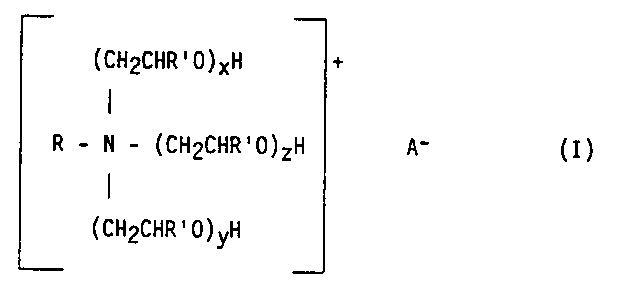 worin
      - R =: einen aliphatischen Kohlenwasserstoffrest mit 6-22 C-Atomen
      - R' =: H oder CH₃
      - x, y, z =: unabhängig voneinander eine Zahl von 1 bis 20 bedeuten, wobei die Summe x+y,z ≥ 3 ist,
      - A⁻ =: Anion bedeuten
   a2) quaternierten Difettsäuretrialkanolaminester der allgemeinen Formel (II) 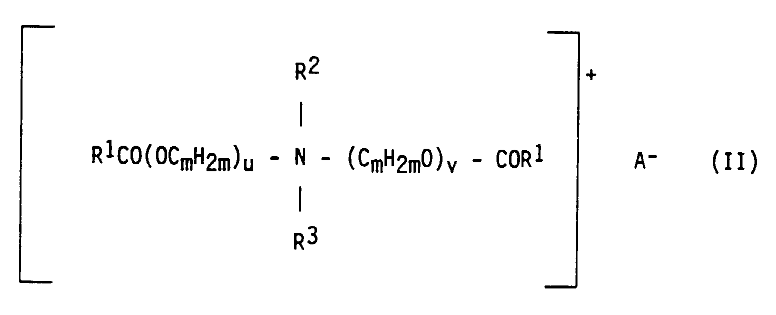 in der R¹CO - eine gesättigte oder ungesättigte Acylgruppe mit 12 bis 22 C-Atomen, R² eine Gruppe R¹CO(OCₘH₂ₘ)_{w} -, eine Alkylgruppe mit 1 bis 4 C-Atomen oder eine Hydroxyalkylgruppe mit 2 bis 4 C-Atomen und R³ eine Alkylgruppe mit 1 bis 4 C-Atomen oder eine Hydroxyalkylgruppe mit 2 bis 4 C-Atomen, m jeweils 2 oder 3, u, v und w jeweils eine Zahl von 1 bis 4 und A⁻ ein Anion ist und
b) einem nicht-ionogenen Emulgator, der ausgewählt ist aus der Gruppe der
   b1) ethoxylierten und/oder propoxylierten Fettsäuren oder Fettsäureester der Formel (III) 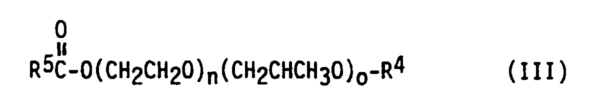 worin
      - R⁵CO =: einen aliphatischen Acylrest mit 6-22 C-Atomen
      - n, o =: eine Zahl von 0 bis 20, wobei die Summe n+o ≥ 1 ist,
      - R⁴ =: H oder einen aliphatischen Kohlenwasserstoffrest mit 1 bis 12 C-Atomen bedeuten
   b2) endgruppenverschlossene Fettalkoholpolyglykolether der allgemeinen Formel (IV)

      R⁷-(CH₂CH₂O)ₚ(CH₂CHCH₃O)_{q}-R⁶ (IV),

      worin
      - R⁷ =: einen aliphatischen Kohlenwasserstoffrest mit 6 bis 22 C-Atomen
      - p, q =: unabhängig voneinander eine Zahl von 0-20, wobei die Summe von p+q ≥ 2 ist,
      - R⁶ =: einen aliphatischen Kohlenwasserstoffrest mit 1 bis 12 C-Atomen bedeuten
als Emulgatoren zur Verbesserung der Kaltwasserdispergierbarkeit von Penta- und/oder Dipentaerythritfettsäurepartialester.

Einzelheiten sind den vorhergehenden Ausführungen zu entnehmen.

### Beispiele

### A) Herstellung der Weichmacher-Konzentrate

**A1)**
   50 g technisches Pentaerythritdistearat wurden aufgeschmolzen und bei 60 °C unter Rühren mit 8,0 g einer etwa 50 gew.-%igen wäßrigen Lösung von dem Phosphorsäuresalz des mit 10 Mol Ethylenoxid ethoxylierten und quaternierten Talgamins sowie mit 12,0 g einer etwa 30 gew.-%igen wäßrigen Lösung von mit 12 Mol Ethylenoxid ethoxylierten Laurinsäuremethylesters sowie mit 2,2 g einer etwa 37 %igen Salzsäure versetzt. Das Produkt wurde auf einer Kühlwalze geschuppt.
**A2)**
   50 g technisches Pentaerythritdistearat wurden aufgeschmolzen und bei 60 °C unter Rühren mit 8,0 g einer etwa 90 gew.-%igen Lösung von Methyl-N,N-bis(acyloxyethyl)-N(2-hydroxyethyl)ammoniummethosulfat in Isopropanol sowie mit 12 g einer etwa 30 gew.-%igen wäßrigen Lösung von mit 12 Mol Ethylenoxid ethoxylierten Laurinsäuremethylesters versetzt. Das Produkt wurde auf einer Kühlwalze geschuppt.
**Vergleichsversuch V1**
   63,5 g technisches Pentaerythritdistearat wurden aufgeschmolzen und bei 60 °C unter Rühren mit 10,4 g eines mit 2 Mol Ethylenoxid ethoxylierten Talgamins, 15,0 g eines mit 2 Mol Ethylenoxid ethoxylierten technischen C₁₀-C₁₈-Fettalkoholgemisches (Kettenverteilung: C₁₀: 0-2 %; C₁₂ 70-75 %; C₁₄: 25-30 %; C₁₆: 0-2 %; C₁₈: 0-1 %) sowie 2,8 g einer etwa 37 %igen Salzsäure versetzt. Das Produkt wurde auf einer Kühlwalze geschuppt.

### B) Prüfung der Kaltwasserlöslichkeit /-dispergierbarkeit

- Methode:: 95 g Wasser wurden vorgelegt und 5 g Schuppen unter Rühren zugesetzt. Nach weiterem Rühren wurde die Kaltwasserdispergierbarkeit nach 10 Minuten geprüft. Anschließend wurde die Qualität der Dispersion nach 16 Stunden ohne weiteres Rühren beurteilt.

| **Ergebnis:** | | |
|---|---|---|
| Beispiel | 10 Minuten Rühren | 16 h Stehen |
| A1 | Schuppen zum großen Teil aufgelöst; feinteilige Dispersion | vollständig aufgelöst; feinteilige Dispersion |
| A2 | Schuppen zum großen Teil aufgelöst; feinteilige Dispersion | vollständig aufgelöst; feinteilige Dispersion |
| V1 | Schuppen nur angequollen; keine Dispersion | Schuppen stärker gequollen; nur teilweise dispergiert |

Die Zusammenstellung zeigt, daß die erfindungsgemäßen Weichmacher-Konzentrate schneller mit und ohne Rühren in Wasser dispergiert werden können.

### C) Weichheitsleistung

### Durchführung:

Ein ca. 25 x 15 cm großer Prüfling aus Baumwollfrotteé wurde gewogen. Danach wurde in einem 1 L-Becherglas mit Leitungswasser von 20 °C die Behandlungsflotte angesetzt. Dabei wurde ein Flottenverhältnis (Gewicht Prüfling zu Flottenmenge) von 1:20 gewählt. Bezogen auf das Gewicht des Prüflings wurden 0,5 % Aktivsubstanz der zu prüfenden Substanz in der Flotte verteilt. Der Prüfling wurde nun 5 Min. leicht, z. B. mit einem Rührstab, in der Flotte bewegt. Anschließend wurde ausgewrungen und 24 Stunden an der Luft getrocknet (Wäscheleine). Dabei sollte der Prüfling in den ersten 2 Stunden 4-mal um jeweils 180 °C gedreht werden, um einseitige Überkonzentrationen zu vermeiden.

### Bewertung:

Zur Beurteilung des Weichgriffs müssen stets frische Standardprüfungen einbezogen werden. Die Beurteilung selbst war eine subjektive (haptische) Prüfung, bei der mindestens 4 Personen die Prüfgewebe abgreifen und in eine Skala von 0 bis 6 einordnen. 0 bedeutet den Griffeindruck des Wasserstandards, d. h. Flotte ohne Wirkstoff und ist gleichbedeutend mit dem schlechtesten Griff. Je besser der Griff, desto höher die Noten, wobei 6 sehr gut ist. Das Ergebnis stellt den Durchschnitt aus den Bewertungen aller Testpersonen dar.

| **Ergebnis:** | |
|---|---|
| Beispiel | Beurteilung |
| A1 | 5,8 |
| A2 | 5,7 |
| V1 | 5,6 |
| Dimethyldistearylammoniumchlorid (Standard) | 5,5 |
| (0 = sehr schlecht, 6 = sehr gut) | |

## Patentansprüche

1. Textile Weichmacher-Konzentrate mit verbesserter Kaltwasserdisperierbarkeit auf Basis von Penta- und/oder Dipentaerythritfettsäurepartialester als Weichmacher, dadurch gekennzeichnet, daß sie zusätzlich eine Emulgatorkombination enthalten aus
a) einer quaternären Stickstoffverbindung ausgewählt aus der Gruppe der
a1) quaternierten alkoxylierten Alkylamine der Formel (I) 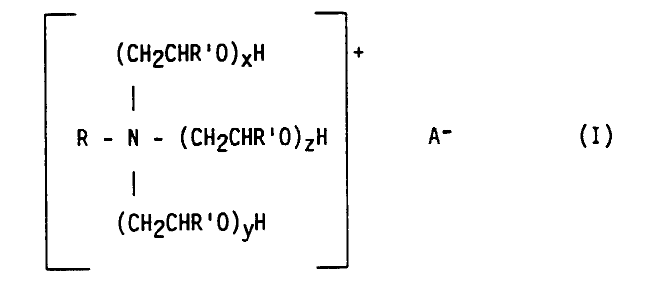 worin
R = einen aliphatischen Kohlenwasserstoffrest mit 6-22 C-Atomen
R' = H oder CH₃
x, y, z = unabhängig voneinander eine Zahl von 1 bis 20 bedeuten, wobei die Summe x+y+z ≥ 3 ist,
A⁻ = Anion bedeuten
a2) quaternierten Fettsäurealkanolaminester der allgemeinen Formel (II) 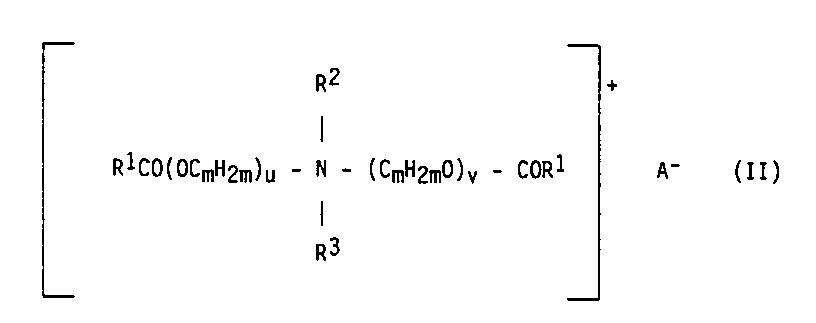 in der R¹CO - eine gesättigte oder ungesättigte Acylgruppe mit 12 bis 22 C-Atomen, R² eine Gruppe R¹CO(OCₘH₂ₘ)_{w} -, eine Alkylgruppe mit 1 bis 4 C-Atomen oder eine Hydroxyalkylgruppe mit 2 bis 4 C-Atomen und R³ eine Alkylgruppe mit 1 bis 4 C-Atomen oder eine Hydroxyalkylgruppe mit 2 bis 4 C-Atomen, m jeweils 2 oder 3, u, v und w jeweils eine Zahl von 1 bis 4 und A⁻ ein Anion ist und
b) einem nicht-ionogenen Emulgator, der ausgewählt ist aus der Gruppe der
b1) ethoxylierten und/oder propoxylierten Fettsäuren oder Fettsäureestern der Formel (III) 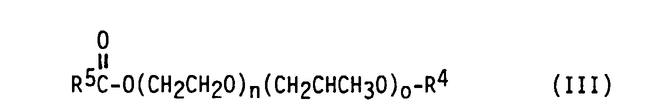 worin
R⁵CO = einen aliphatischen Acylrest mit 6 bis 22 C-Atomen
n, o = unabhängig voneinander eine Zahl von 0 bis 20, wobei die Summe n+o ≥ 1 ist,
R⁴ = H oder einen aliphatischen Kohlenwasserstoffrest mit 1 bis 12 C-Atomen bedeuten
b2) endgruppenverschlossene Fettalkoholpolyglykolether der allgemeinen Formel (IV)
R⁷-(CH₂CH₂O)ₚ(CH₂CHCH₃O)_{q}-R⁶ (IV),
worin
R⁷ = einen aliphatischen Kohlenwasserstoffrest mit 6 bis 22 C-Atomen
p, q = unabhängig voneinander eine Zahl von 0-20, wobei die Summe von p+q ≥ 2 ist,
R⁶ = einen aliphatischen Kohlenwasserstoffrest mit 1 bis 12 C-Atomen bedeuten.

2. Textile Weichmacher-Konzentrate nach Anspruch 1, dadurch gekennzeichnet, daß das Anion A⁻ in Formel (I) für einen entsprechenden Äquivalentanteil des Phosphats und in (II) für ein Halogenid, Methosulfat oder Methophosphat steht.

3. Textile Weichmacher-Konzentrate nach einem der Ansprüche 1 oder 2, dadurch gekennzeichnet, daß der nicht-ionogene Emulgator ein ethoxylierter und/oder propoxylierter Fettsäurerest ist, wobei in Formel (III) R⁵CO für einen aliphatischen Acylrest mit 12 bis 22 C-Atomen, n eine Zahl von 5 bis 15, o die Zahl 0 und R⁴ H oder einen Alkylrest mit 1 bis 4 C-Atomen bedeutet.

4. Textile Weichmacher-Konzentrate nach einem der Ansprüche 1 bis 3, dadurch gekennzeichnet, daß der nicht-ionogene Emulgator ein endgruppenverschlossener Fettalkoholpolyglykolether ist, wobei in Formel (IV) R⁷ für einen aliphatischen Kohlenwasserstoffrest mit 12 bis 22 C-Atomen, p eine Zahl von 2 bis 8 und q eine Zahl von 0 bis 5 ist, und R⁶ für einen Alkylrest mit 1 bis 4 C-Atomen steht.

5. Textile Weichmacher-Konzentrate nach einem der Ansprüche 1 bis 4, dadurch gekennzeichnet, daß die Emulgatorkombination die nicht-ionischen Emulgatoren im Gewichtsverhältnis zu den quaternären Stickstoffverbindungen von 1:1 bis 3:1, vorzugsweise 1,5:1 bis 2:1 enthält.

6. Textile Weichmacher-Konzentrate nach einem der Ansprüche 1 bis 5, dadurch gekennzeichnet, daß die Weichmacher Pentaerythritdifettsäureester von Fettsäuren mit 16-22 C-Atomen sind, vorzugsweise reines oder technisches Pentaerythritdistearat.

7. Textile Weichmacher-Konzentrate nach einem der Ansprüche 1 bis 6, dadurch gekennzeichnet, daß sie kein zusätzliches Wasser enthalten.

8. Textile Weichmacher-Konzentrate nach einem der Ansprüche 1 bis 7, dadurch gekennzeichnet, daß sie
70 bis 95 Gew.-% Penta- und/oder Dipentaerythritfettsäurepartialester
5 bis 30 Gew.-% Emulgatorkombination
0 bis 20 Gew.-% übliche Hilfsstoffe
enthalten.

9. Verfahren zur Herstellung von textilen Weichmacher-Konzentraten, dadurch gekennzeichnet, daß Penta- und/oder Dipentaerythritfettsäurepartialester als Weichmacher zunächst aufgeschmolzen und mit einer Emulgatorkombination nach Anspruch 1 versetzt werden.

10. Verwendung einer Kombination aus
a) einer quaternären Stickstoffverbindung ausgewählt aus der Gruppe der
a1) quaternierten alkoxylierten Alkylamine der Formel (I) 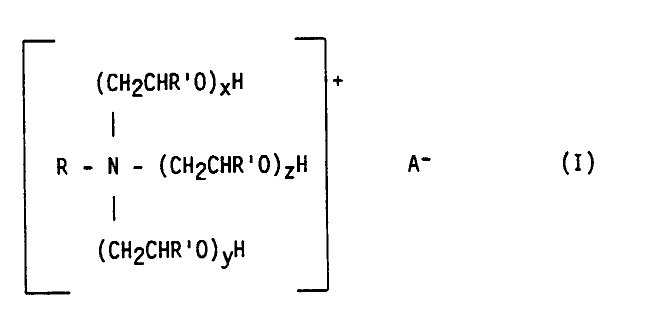 worin
R = einen aliphatischen Kohlenwasserstoffrest mit 6-22 C-Atomen
R' = H oder CH₃
x, y, z = unabhängig voneinander eine Zahl von 1 bis 20 bedeuten, wobei die Summe x+y,z ≥ 3 ist,
A⁻ = Anion bedeuten
a2) quaternierten Fettsäurealkanolaminester der allgemeinen Formel (II) 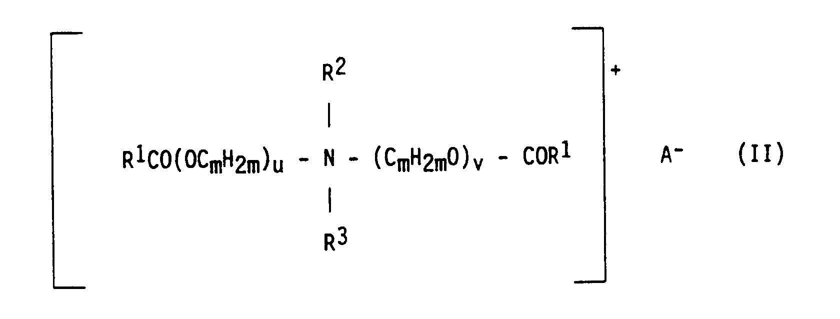 in der R¹CO - eine gesättigte oder ungesättigte Acylgruppe mit 12 bis 22 C-Atomen, R² eine Gruppe R¹CO(OCₘH₂ₘ)_{w} -, eine Alkylgruppe mit 1 bis 4 C-Atomen oder eine Hydroxyalkylgruppe mit 2 bis 4 C-Atomen und R³ eine Alkylgruppe mit 1 bis 4 C-Atomen oder eine Hydroxyalkylgruppe mit 2 bis 4 C-Atomen, m jeweils 2 oder 3, u, v und w jeweils eine Zahl von 1 bis 4 und A- ein Anion ist und
b) einem nicht-ionogenen Emulgator, der ausgewählt ist aus der Gruppe der
b1) ethoxylierten und/oder propoxylierten Fettsäuren oder Fettsäureestern der Formel (III) 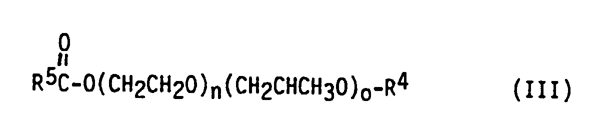 worin
R⁵CO = einen aliphatischen Acylrest mit 6-22 C-Atomen
n, o = eine Zahl von 0 bis 20, wobei die Summe n+o > 1 ist,
R⁴ = H oder einen aliphatischen Kohlenwasserstoffrest mit 1 bis 12 C-Atomen bedeuten
b2) endgruppenverschlossene Fettalkoholpolyglykolether der allgemeinen Formel (IV)
R⁷-(CH₂CH₂O)ₚ(CH₂CHCH₃O)_{q}-R⁶ (IV),
worin
R⁷ = einen aliphatischen Kohlenwasserstoffrest mit 6 bis 22 C-Atomen
p, q = unabhängig voneinander eine Zahl von 0-20, wobei die Summe von p+q > 2 ist,
R⁶ = einen aliphatischen Kohlenwasserstoffrest mit 1 bis 12 C-Atomen bedeuten
als Emulgatoren zur Verbesserung der Kaltwasserdispergierbarkeit von Penta- und/oder Dipentaerythritfettsäurepartialester.

## Claims

1. Textile softener concentrates with improved dispersibility in cold water based on pentaerythritol and/or dipentaerythritol fatty acid partial esters as softeners, characterized in that they additionally contain an emulsifier combination of
a) a quaternary nitrogen compound selected from the group of
a1 ) quaternized alkoxylated alkylamines corresponding to formula (I): 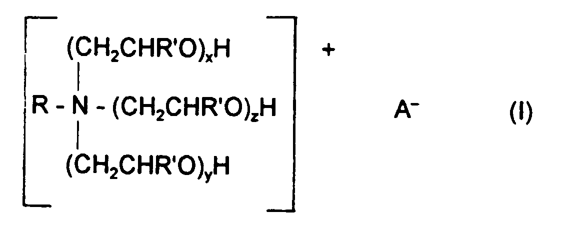 in which
R is an aliphatic hydrocarbon radical containing 6 to 22 carbon atoms,
R' is H or CH₃,
x, y, and z independently of one another represent a number of 1 to 20, the sum of x+y+z being ≥ 3,
A is an anion,
a2) quaternized fatty acid alkanolamine esters corresponding to general formula (II): 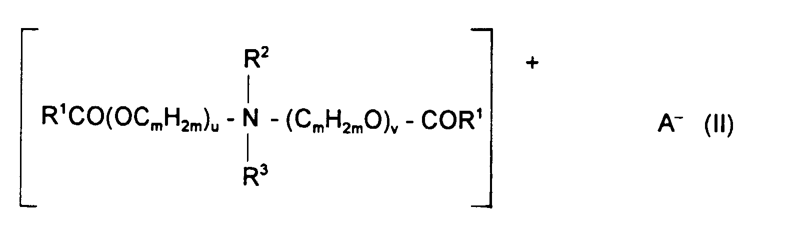 in which R¹CO is a saturated or unsaturated acyl group containing 12 to 22 carbon atoms, R² is a group R¹CO(OCₘH₂ₘ)_{w}-, an alkyl group containing 1 to 4 carbon atoms or a hydroxyalkyl group containing 2 to 4 carbon atoms and R³ is an alkyl group containing 1 to 4 carbon atoms or a hydroxyalkyl group containing 2 to 4 carbon atoms, m is the number 2 or 3, u, v and w are each a number of 1 to 4 and A⁻ is an anion, and
b) a nonionic emulsifier selected from the group of
b1) ethoxylated and/or propoxylated fatty acids or fatty acid esters corresponding to formula (III): 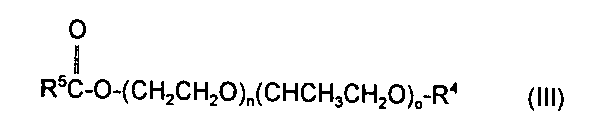 in which
R⁵CO is an aliphatic acyl radical containing 6 to 22 carbon atoms,
n and o independently of one another represent a number of 0 to 20, the sum of n+o being ≥ 1,
R⁴ is H or an aliphatic hydrocarbon radical containing 1 to 12 carbon atoms,
b2) end-capped fatty alcohol polyglycol ethers corresponding to general formula (IV):
R⁷-(CH₂CH₂O)ₚ(CHCH₃CH₂O)_{q}-R⁶ (IV)
in which
R⁷ is an aliphatic hydrocarbon radical containing 6 to 22 carbon atoms,
p and q independently of one another are numbers of 0 to 20, the sum of p+q being ≥ 2,
R⁶ is an aliphatic hydrocarbon radical containing 1 to 12 carbon atoms.

2. Textile softener concentrates as claimed in claim 1, characterized in that the anion A in formula (I) stands for a corresponding equivalent of the phosphate and, in formula (II), for a halide, methosulfate or methophosphate.

3. Textile softener concentrates as claimed in claim 1 or 2, characterized in that the nonionic emulsifier is an ethoxylated and/or propoxylated fatty acid, R⁵CO in formula (III) being an aliphatic acyl radical containing 12 to 22 carbon atoms, n being a number of 5 to 15, o being the number 0 and R⁴ being H or an alkyl radical containing 1 to 4 carbon atoms.

4. Textile softener concentrates as claimed in any of claims 1 to 3, characterized in that the nonionic emulsifier is an end-capped fatty alcohol polyglycol ether, R⁷ in formula (IV) being an aliphatic hydrocarbon radical containing 12 to 22 carbon atoms, p being a number of 2 to 8, q being a number of 0 to 5 and R⁶ being an alkyl radical containing 1 to 4 carbon atoms.

5. Textile softener concentrates as claimed in any of claims 1 to 4, characterized in that the emulsifier combination contains the nonionic emulsifiers in a ratio by weight to the quaternary nitrogen compounds of 1:1 to 3:1 and preferably 1.5:1 to 2:1.

6. Textile softener concentrates as claimed in any of claims 1 to 5, characterized in that the softeners are pentaerythritol difatty acid esters of fatty acids containing 16 to 22 carbon atoms, preferably pure or technical pentaerythritol distearate.

7. Textile softener concentrates as claimed in any of claims 1 to 6, characterized in that they do not contain any additional water.

8. Textile softener concentrates as claimed in any of claims 1 to 7, characterized in that they contain
| | |
|---|---|
| 70 to 95% by weight | of pentaerythritol and/or dipentaerythritol fatty acid partial ester, |
| 5 to 30% by weight | of the emulsifier combination, |
| 0 to 20% by weight | of typical auxiliaries. |

9. A process for the production of textile softener concentrates, characterized in that pentaerythritol and/or dipentaerythritol fatty acid partial esters as softeners are first melted and an emulsifier combination according to claim 1 is then added.

10. The use of a combination of
a) a quaternary nitrogen compound selected from the group of
a1) quatemized alkoxylated alkylamines corresponding to formula (I): 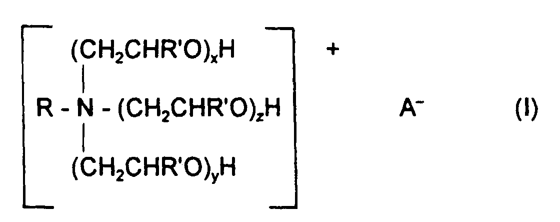 in which
R is an aliphatic hydrocarbon radical containing 6 to 22 carbon atoms,
R' is H or CH₃,
x, y, and z independently of one another represent a number of 1 to 20, the sum of x+y+z being 3,
A is an anion,
a2) quaternized fatty acid alkanolamine esters corresponding to general formula (II): 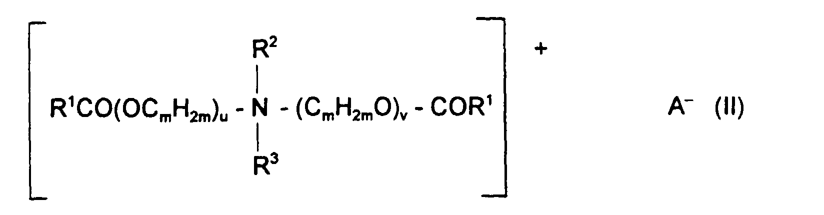 in which R¹CO is a saturated or unsaturated acyl group containing 12 to 22 carbon atoms, R² is a group R¹CO(OCₘH₂ₘ)_{w}-, an alkyl group containing 1 to 4 carbon atoms or a hydroxyalkyl group containing 2 to 4 carbon atoms and R³ is an alkyl group containing 1 to 4 carbon atoms or a hydroxyalkyl group containing 2 to 4 carbon atoms, m is the number 2 or 3, u, v and w are each a number of 1 to 4 and A⁻ is an anion, and
b) a nonionic emulsifier selected from the group of
b1) ethoxylated and/or propoxylated fatty acids or fatty acid esters corresponding to formula (III): 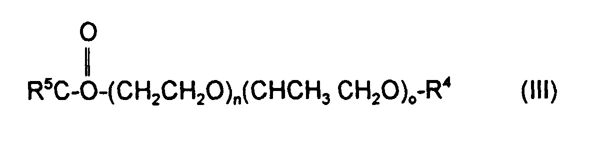 in which
R⁵CO is an aliphatic acyl radical containing 6 to 22 carbon atoms,
n and o independently of one another represent a number of 0 to 20, the sum of n+o being ≥ 1,
R⁴ is H or an aliphatic hydrocarbon radical containing 1 to 12 carbon atoms,
b2) end-capped fatty alcohol polyglycol ethers corresponding to general formula (IV):
R⁷-(CH₂CH₂O)ₚ(CHCH₃ CH₂O)_{q}-R⁶ (IV)
in which
R⁷ is an aliphatic hydrocarbon radical containing 6 to 22 carbon atoms,
p and q independently of one another are numbers of 0 to 20, the sum of p+q being ≥ 2,
R⁶ is an aliphatic hydrocarbon radical containing 1 to 12 carbon atoms,
as emulsifiers for improving the dispersibility of pentaerythritol and/or dipentaerythritol fatty acid partial esters in cold water.

## Revendications

1. Concentrés assouplissants pour textiles avec une possibilité de dispersion dans l'eau froide améliorée à base d'esters partiels d'acides gras de penta et/ou de dipentaérythritol comme assouplissant,
caractérisés en ce qu'
ils contiennent en plus d'une combinaison d'émulsionnants :
a) un composé d'azote quaternaire choisi dans le groupe des
a1) alkylamines quaternées alcoxylées de la formule (I) : 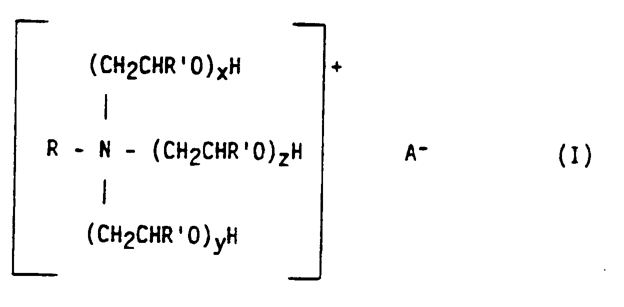 dans laquelle
R = un radical d'hydrocarbure aliphatique avec 6-22 atomes de C
R' = H ou CH₃
x, y, z = indépendamment l'un de l'autre un nombre de 1 à 20, dans lequel la somme x + y + z ≥ 3,
A⁻ = un anion.
a2) des esters d'amines d'alcanol et d'acide gras quaternaires de la formule (II): 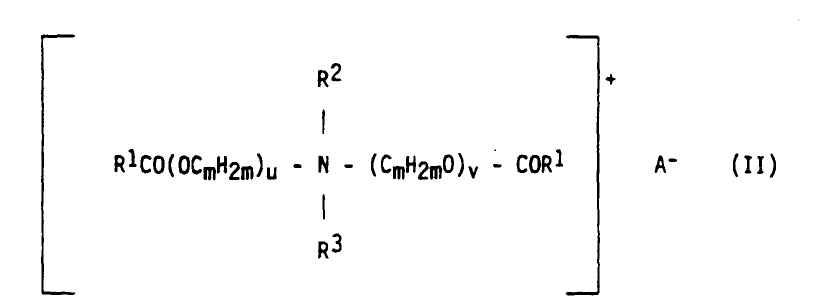 dans laquelle R¹CO est un groupe acyle saturé ou non ayant 12 à 22 atomes de C, R² est un groupe R¹CO(OC_{M}H₂ₘ)_{w}⁻ un groupe alkyle avec 1 à 4 atomes de C ou un groupe hydroxyalkyle avec 2 à 4 atomes de C et R³ un groupe alkyle avec 1 à 4 atomes de C ou un groupe hydroxyalkyle avec 2 à 4 atomes de C, m est respectivement 2 ou 3, u, v et w sont respectivement un nombre de 1 à 4 et A⁻ est un anion.
b) un émulsionnant non ionogène, qui est choisi dans le groupe des
b1) acides gras ou esters d'acides gras éthoxylés et/ou propoxylés de la formule (III) : 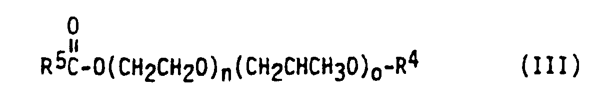 dans laquelle :
R⁵CO représente un radical acyle aliphatique avec 6 à 22 atomes de C,
n, o représentent indépendamment l'un de l'autre un nombre de 0 à 20, la somme n + o ≥ 1,
R⁴ représente H ou un radical d'hydrocarbure aliphatique ayant 1 à 12 atomes de C.
b2) des polyglycoléthers d'alcool gras fermés par des groupes terminaux de formule générale (IV) :
R⁷-(CH₂CH₂O)ₚ(CH₂CHCH₃O)_{q}-R⁶ (IV),
dans laquelle
R⁷ représente un radical d'hydrocarbure aliphatique avec 6 à 22 atomes de C,
p, q représentent indépendamment l'un de l'autre 6 un nombre de 0 à 20, la comme de p + q ≥ 2,
R⁶ représente un radical d'hydrocarbure aliphatique avec 1 à 12 atomes de C.

2. Concentrés assouplissants pour textiles, selon la revendication 1,
caractérisés en ce que
l'anion A⁻ de la formule (I) représente une proportion d'équivalent adéquate du phosphate et dans la formule (II) un halogénure, un méthosulfate ou un méthophosphate.

3. Concentrés assouplissants pour textiles, selon une des revendications 1 ou 2,
caractérisés en ce que
l'émulsionnant non ionogène est un radical d'acide gras éthoxylé et/ou propoxylé, dans la formule (III) R⁵CO représentant un radical acyle aliphatique avec 12 à 22 atomes de C, n un nombre de 5 à 15, o le nombre 0 et R⁴ H ou un radical alkyle ayant 1 à 4 atomes de C.

4. Concentrés assouplissants pour textiles, selon une des revendications 1 à 3,
caractérisés en ce que
l'émulsionnant non ionogène est un polyglycoléther d'alcool gras fermé par des groupes terminaux, dans la formule (IV) R⁷ représentant un radical d'hydrocarbure aliphatique avec 12 à 22 atomes de C, p un nombre de 2 à 8, q un nombre de 0 à 5 et R⁶ un radical alkyle ayant de 1 à 4 atomes de C.

5. Concentrés assouplissants pour textiles, selon une des revendications 1 à 4,
caractérisés en ce que
la combinaison d'émulsionnants contient les émulsionnants non ionogènes dans le rapport de poids aux composés d'azote quaternaires de 1:1 à 3:1, de préférence de 1,5:1 à 2:1.

6. Concentrés assouplissants pour textiles, selon une des revendications 1 à 5,
caractérisés en ce que
les assouplissants sont des diesters d'acide gras et de pentaérythritol d'acide gras ayant 16 à 22 atomes de C, de préférence du distéarate de pentaérythritol pur ou technique.

7. Concentrés d'assouplissants pour textiles, selon une des revendications 1 à 6,
caractérisés en ce qu'
ils ne contiennent pas d'eau additionnelle.

8. Concentrés d'assouplissants pour textiles, selon une des revendications 1 à 7,
caractérisés en ce qu'
ils contiennent :
70 à 95 % en poids d'esters partiels d'acides gras de penta et/ou de dipentaérythritol,
5 à 30 % en poids des combinaisons d'émulsionnants décrits et
0 à 20 % en poids de matières auxiliaires usuelles.

9. Procédé de fabrication de concentrés d'assouplissants pour textiles,
caractérisé en ce que
des esters partiels d'acide gras de penta et/ou de pentaérythritol comme assouplissants sont d'abords fondus et mélangés avec une combinaison d'émulsionnants selon la revendication 1.

10. Utilisation d'une combinaison de
a) un composé d'azote quaternaire choisi dans le groupe des
a1) alkylamines quaternées alcoxylées de la formule (I) : 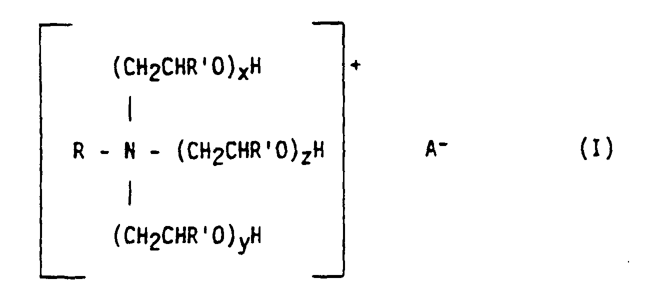 dans laquelle
R = un radical d'hydrocarbure aliphatique avec 6-22 atomes de C
R' = H ou CH₃
x, y, z = indépendamment l'un de l'autre un nombre de 1 à 20, dans lequel la somme x + y + z ≥ 3,
A⁻ = un anion.
a2) des esters d'amines d'alcanol et d'acide gras quaternaires de la formule (II) : 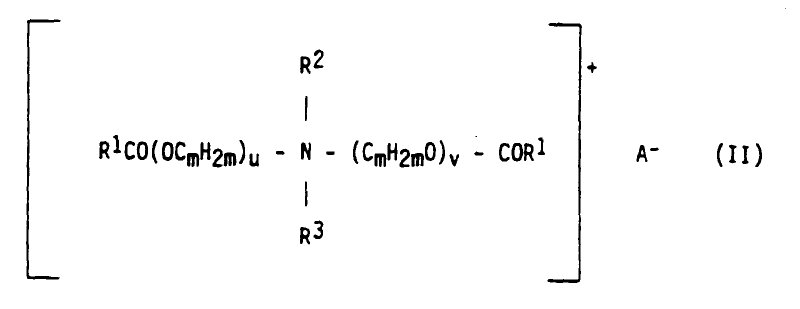 dans laquelle R¹CO est un groupe acyle saturé ou non ayant 12 à 22 atomes de C, R² est un groupe R¹CO(OC_{M}H₂ₘ)_{w}⁻ un groupe alkyle avec 1 à 4 atomes de C ou un groupe hydroxyalkyle avec 2 à 4 atomes de C et R³ un groupe alkyle avec 1 à 4 atomes de C ou un groupe hydroxyalkyle avec 2 à 4 atomes de C, m est respectivement 2 ou 3, u, v et w sont respectivement un nombre de 1 à 4 et A⁻ est un anion.
b) un émulsionnant non ionogène, qui est choisi dans le groupe des
b1) acides gras ou esters d'acides gras éthoxylés et/ou propoxylés de la formule (III) : 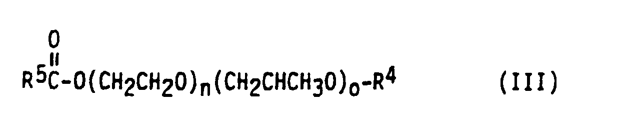 dans laquelle :
R⁵CO représente un radical acyle aliphatique avec 6 à 22 atomes de C,
n, o représentent indépendamment l'un de l'autre un nombre de 0 à 20, la somme n + o ≥ 1,
R⁴ représente H ou un radical d'hydrocarbure aliphatique ayant 1 à 12 atomes de C.
b2) des polyglycoléthers d'alcool gras fermés par des groupes terminaux de formule générale (IV) :
R⁷-(CH₂CH₂O)ₚ(CH₂CHCH₃O)_{q}-R⁶ (IV),
dans laquelle
R⁷ représente un radical d'hydrocarbure aliphatique avec 6 à 22 atomes de C,
p, q représentent indépendamment l'un de l'autre un nombre de 0 à 20, la comme de p + q ≥ 2,
R⁶ représente un radical d'hydrocarbure aliphatique avec 1 à 12 atomes de C,
comme émulsionnants pour l'amélioration de la possibilité de dispersion dans l'eau froide d'esters partiels d'acide gras de penta- et/ou dipentaérythritol.
